# EUROPEAN PATENT APPLICATION

(11) **EP 2 594 206 A1**
(43) Date of publication of application: **22.05.2013**
(21) Application number: 11806212.4
(22) Date of filing: 14.07.2011
(51) Int. Cl.: A61B 17/12

(54) **INFLATION-DEFLATION DEVICE FOR TOURNIQUET**

(30) Priority: 14.07.2010 CN 201020264109 U
(71) Applicant: Lepu Medical Technology (Beijing) Co., Ltd., Changping Tech. Zone Beijing 102200 (CN)
(72) Inventor: LIU, Wanbing, Beijing 102200 (CN); LI, Shuxin, Beijing 102200 (CN)
(74) Representative: HOFFMANN EITLE
(86) International application number: PCT/CN2011/001164
(87) International publication number: WO 2012/006870

(57) **Abstract**

An inflation-deflation device for tourniquet comprising: a barrel (11) and a plunger (12) fitting tightly within the barrel (11). A connecting rod (13) is fixed at one end of the plunger (12). The barrel (11) is fixedly connected at its bottom to a short tube (10). The short tube (10) is connected to the interior space of the barrel (11). The connecting rod (13) having a cruciform cross-section structure, a baffle (16) is perpendicularly fixed to the two sides opposite each other from the central axis of the structure. Connected on the barrel (11) is a clasp (15), the clasp (15) is fixedly connected to the barrel (11) and extends into the interior surface of the barrel (11). The inflation-deflation device has a distinctively designed inflation hole connection tip, to prevent confusion with a standard Luer connection of an ordinary liquid syringe, and also has a distinctively designed deflation limit, to control effectively the volume of gas discharged in a single deflation, and to prevent mishandling.

## Description

### Technical Field

The present application relates to the field of medical instrument, and in particular relates to an inflation-deflation device for a compression tourniquet for radial artery.

### Background Art

A compression tourniquet is used in surgery to be intervened into the radial artery for compression hemostasis. At present, the matching inflation-deflation device used with the compression tourniquet is usually a common 20ml syringe or a gas syringe instrument similar to the 20ml syringe. When using this kind of gas syringe instrument as the inflation-deflation device for inflatable hemostasis compressor, it is unable to effectively and accurately control the amount of gas that is discharged during the operation, which results in inconvenient manipulation. In addition, because the syringe is generally in a standard Luer structure, in operation, the surgeon is likely to confuse the one-way valve with the extended canal of sheathing canal, thus injects the gas into the sheathing canal by mistake, thereby resulting in medical accident.

### Summary of the Invention

### Technical Problem to be Solved

The technical problem to be solved by this invention is to provide an inflation-deflation device which can be quickly and easily connected to the compression tourniquet, can keep effective seal during the inflation and deflation operations, and is able to control accurately the amount of gas that is discharged during the operation according to the operating requirements of product. The said inflation-deflation device can prevent gas from being injected into the sheathing canal by mistake and thereby can avoid medical accident.

### Technical Solution

To achieve the above-mentioned object, the technical solution of this invention provides an inflation-deflation device for tourniquet, comprising a barrel and a plunger fitting tightly within the barrel. A connecting rod is fixed at one end of the plunger. Wherein, the barrel is fixedly connected at its bottom to a short tube. The short tube is communicated with the interior space of the barrel. The connecting rod has a cruciform column structure, and baffles are perpendicularly fixed to the two opposite sides of the central axis of the cruciform column. What is connected on the barrel is a clasp, the clasp is fixedly connected to the barrel and extends into the interior surface of the barrel.

Wherein, the outer surface of the short tube is in a shape of step, and the big end of the step is connected to the bottom of the barrel.

Wherein, the outer surface of the short tube is in a shape of three-stage step and the step is a tapered ladder.

Wherein, the outer surface of the barrel is provided with scale.

Wherein, the diameter of the slimmest portion of the tapered ladder of the short tube ranges from 1mm to 4 mm.

Wherein, the inner diameter of the short tube ranges from 0.5mm to 0.8 mm.

Wherein, the diameter of the second biggest end of the short tube ranges from 1.5mm to 6mm.

Wherein, the end of the barrel that is far away from the short tube is extruding outwardly along the radial direction to form a protruding ring.

### Beneficial Effect

The present application provides an inflation-deflation device with distinctively designed connector end for inflation inlet, to prevent being confused with a standard Luer connector of an ordinary liquid syringe and to keep an effective seal during the inflation and deflation operations; meanwhile it has a distinctively designed deflation limit, to control effectively and accurately the amount of gas that is discharged during a single deflation, and to prevent mishandling. At the same time, the said inflation-deflation device can prevent gas from being injected into the sheathing canal by mistake and thereby can avoid medical accident.

### Brief Description of the Drawings

Fig. 1 is a schematic view showing a cut-away structure of the inflation-deflation device for tourniquet according to the present invention;
Fig. 2 is another schematic view showing a cut-away structure of the inflation-deflation device for tourniquet according to the present invention;
Fig. 3 is an enlarged view of the short tube portion of the inflation-deflation device for tourniquet according to the present invention;
Fig. 4 is a cut-away view of the clasp portion of the inflation-deflation device for tourniquet according to the present invention;
Fig. 5 is a connection view showing the inflation-deflation device for tourniquet according to the present invention in use connected to the compression tourniquet;
Fig. 6 is a view showing the clasp and the baffle of the inflation-deflation device for tourniquet in a connected state.

Reference numbers in drawings:
10: short tube
11: barrel
12: plunger
13: connecting rod
14: protruding ring
15: clasp
16: baffle

### Specific Mode for Carrying Out the Invention

Specific embodiments of the present invention will be described in details below with reference to the accompanying drawings. Such embodiments are presented herein for description purpose only, but not for limiting the scope of the present application.

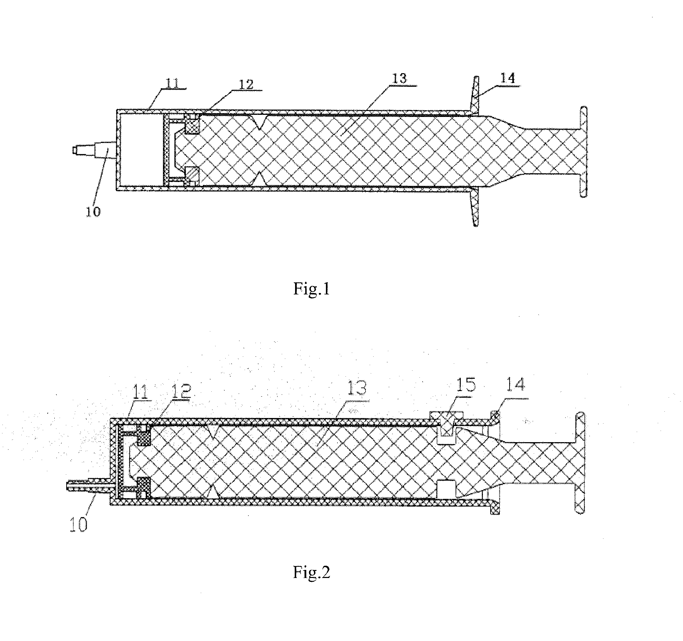

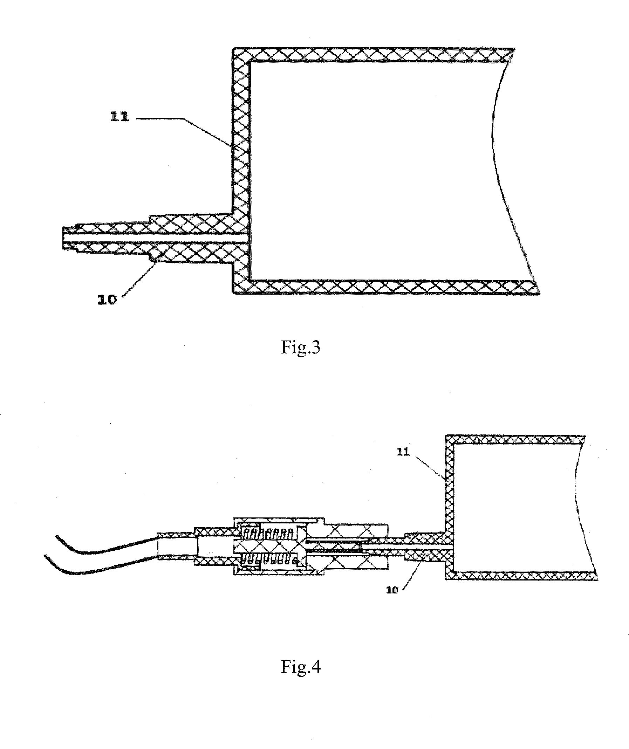

As shown in Fig. 1-2, an inflation-deflation device for tourniquet according to one embodiment of this invention comprises: a barrel 11 and a plunger 12 fitting tightly within the barrel 11. A connecting rod 13 is fixed at one end of the plunger 12. The barrel 11 is fixedly connected at its bottom to a short tube 10. The short tube 10 is communicated with the interior space of the barrel 11. The connecting rod 13 has a cruciform column structure, and baffles 16 are perpendicularly fixed to the two opposite sides of the central axis of the cruciform column. What is connected on the barrel 11 is a clasp 15, the clasp 15 is fixedly connected to the barrel 11 and extends into the interior surface of the barrel 11 (as shown in Fig. 4). When the connecting rod 13 is retracting, the clasp 15 comes into contact with the baffle 16 for obstructing the retraction of the connecting rod 13.

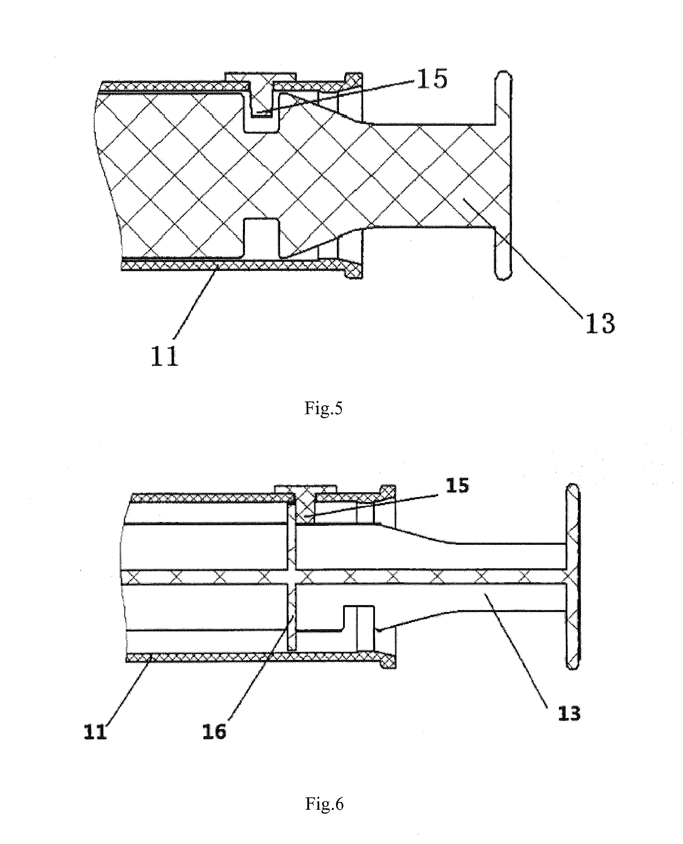

In order to prevent a gas leakage from the tourniquet when connected with the compression tourniquet, the outer surface of the short tube 10 is in a shape of step; preferably, the outer surface of the short tube 10 is in a shape of three-stage tapered step (as shown in Fig. 3). The big end of the step is connected to the bottom of the barrel 11. As shown in Fig. 5, the connector end of the short tube 10 exactly comes into contact with the plunger of the one-way valve on the compression tourniquet; at this time, the second-stage tapered step segment at the connector end of the short tube 10 in the inflation-deflation device has been attached to the inner wall of the connector of the one-way valve, thus pushing inwardly to slightly rotate the inflation-deflation device. The short tube 10 at the tip of the inflation inlet compresses the plunger of the one-way valve to open the inflation-deflation passage; meanwhile the second-stage tapered step segment at the connector end of the short tube 10 of the inflation inlet is tightly attached to the inner wall of the connector of the one-way valve, so as to prevent the gas from leakage. Preferably, the diameter of the slimmest portion of the tapered step of the short tube 10 ranges from 1mm to 4 mm. Preferably, the diameter of the second biggest end of the short tube 10 ranges from 1.5mm to 6mm (the diameter can be configured according to the size of the inner wall of the connector of the one-way valve of the tourniquet). Preferably, the inner diameter of the short tube ranges from 0.5mm to 0.8 mm.

For the purpose of convenient grip, the end of the barrel 11 that is far away from the short tube is extruding outwardly along the radial direction to form a protruding ring 14. During operation, the connecting rod 13 is pulled outwardly. When the baffle 16 on the connecting rod 13 reaches the position of the clasp 15 (as shown in Fig. 6), it obstructs the movement of the connecting rod 13, so as to achieve a precise control of the amount of gas that is filled and discharged. The outer surface of the barrel 11 can be provided with scale according to actual needs.

The inflation-deflation device for tourniquet described herein has functions of both deflation and inflation. When the inflation function is executed, the connecting rod 13 is rotated to locate the limiting clasp 15 in the inflation function area (i.e. its position is deviated from that of the baffle 16) and the connecting rod 13 is pulled to reach the corresponding scale on the barrel 11, and hence the amount of gas that is filled can be ensured. When the deflation function is executed, the plunger 12 is pushed to the end portion and the connecting rod 13 is rotated to locate the limiting clasp 15 in the deflation function area (namely when the connecting rod 13 is pulled, it will drive the baffle 16 on the connection rod 13 to move to the position of clasp 15, then the baffle 16 is blocked by the clasp 15 and thus the amount of gas that is discharged can be ensured). The amount of gas that is discharged can be set as stipulated in the product specification of the hemostasis compressor.

### Industrial applicability

The inflation-deflation device provided by this invention has a distinctively designed connector end for inflation inlet, to prevent from be confused with a standard Luer connector of an ordinary liquid syringe, and also has a distinctively designed deflation limit, to control effectively and accurately the amount of gas that is discharged during a single deflation, and to prevent mishandling. At the same time, the said inflation-deflation device can prevent gas from being injected into the sheathing canal by mistake and thereby can avoid medical accident.

## Claims

1. An inflation-deflation device for tourniquet, comprising: a barrel and a plunger fitting tightly within the barrel, a connecting rod is fixed at one end of the plunger, **characterized in that**,
the barrel is fixedly connected at its bottom to a short tube; the short tube is connected to the interior space of the barrel; the connecting rod has a cruciform column structure, and a baffle is perpendicularly fixed to the two opposite sides of the central axis of the cruciform column; what is connected on the barrel is a clasp, the clasp is fixedly connected to the barrel and extends into the interior surface of the barrel.

2. The inflation-deflation device according to claim 1, **characterized in that**, the outer surface of the short tube is in a shape of step, and the big end of the step is connected to the bottom of the barrel.

3. The inflation-deflation device according to claim 1 or 2, **characterized in that**, the outer surface of the short tube is in a shape of three-stage step, and the step is a tapered ladder.

4. The inflation-deflation device according to claim 3, **characterized in that**, the outer surface of the short tube is provided with scale.

5. The inflation-deflation device according to claim 4, **characterized in that**, the diameter of the slimmest portion of the tapered ladder of the short tube ranges from 1mm to 4 mm.

6. The inflation-deflation device according to claim 5, **characterized in that** the inner diameter of the short tube ranges from 0.5mm to 0.8 mm.

7. The inflation-deflation device according to claim 6, **characterized in that** the diameter of the second biggest end of the short tube ranges from 1.5mm to 6mm.

8. The inflation-deflation device according to claim 7, **characterized in that**, the end of the barrel that is far away from the short tube is extruding outwardly along the radial direction to form a protruding ring.
